# EUROPEAN PATENT SPECIFICATION

(11) **EP 2 838 494 B1**
(45) Date of publication and mention of the grant of the patent: **31.01.2018**
(21) Application number: 13725500.6
(22) Date of filing: 18.04.2013
(51) Int. Cl.: A61K 8/31, A61K 8/34, A61K 8/37, A61K 8/92, A61K 8/85, A61Q 19/10, A61Q 19/00

(54) **IMPROVING SKIN APPEARANCE WITH INCREASE IN SKIN CHROMA**
VERBESSERUNG DES AUSSEHENS DER HAUT MIT VERSTÄRKUNG DER HAUTFARBE
AMÉLIORATION DE L'ASPECT DE LA PEAU AVEC AUGMENTATION DE LA SATURATION DE LA PEAU

(30) Priority: 20.04.2012 US 201261635886 P
(43) Date of publication of application: 25.02.2015
(73) Proprietor: The Procter & Gamble Company, Cincinnati, OH 45202 (US)
(72) Inventor: STELLA, Qing, Cincinnati, Ohio 45202 (US); MOTLEY, Curtis, Bobby, Cincinnati, Ohio 45202 (US); ARREDONDO, Victor, Manuel, Cincinnati, Ohio 45202 (US); GARZA, Cynthia, Ann, Cincinnati, Ohio 45202 (US)
(74) Representative: Kremer, Véronique Marie Joséphine
(86) International application number: PCT/US2013/037126
(87) International publication number: WO 2013/158851

(56) References cited:
- EP-A2- 0 552 024
- WO-A1-96/25144
- WO-A2-2009/130144
- WO-A2-2011/156672
- US-A1- 2006 079 422
- MICHAEL STARCH: "New Cosmetic Ingredients Based on Soybean Oil", IP.COM JOURNAL, IP.COM INC., WEST HENRIETTA, NY, US, 15 June 2007 (2007-06-15), XP013120951, ISSN: 1533-0001

## Description

### TECHNICAL FIELD

The present disclosure generally relates to methods for increasing skin chroma, enhancing skin hydration, decreasing dry skin grade, and improving overall skin health.

### BACKGROUND

Cleansing the skin is an activity that has been done for millennia. Skin cleansing and methods therefor have involved the utilization of soaps, body washes, and other personal cleansing compositions, which can provide additional benefits to consumers. Healthy skin appearance, for example, is a highly desired benefit that consumers seek from such personal cleansing compositions. Healthy skin typically has a low shine and a high sheen to provide a natural color or a high chroma. Chroma is a parameter that measures skin color saturation. A color having a high chroma is vivid, and free of white, while a color having a low chroma is dull and washed-out. Deep wrinkles and dry skin flakes, which can result from dehydrated epidermis, can produce regions with high contrast to skin surface, block natural color, and scatter light in different directions generating white spots, thereby negatively affecting such properties associated with a healthy skin appearance. Thus, it is desirable to provide methods for increasing skin chroma through enhancing skin hydration, decreasing dry skin grade, and improving overall skin health. Patent document WO 2011/156672 discloses a composition for treating the skin comprising two phases and includes a hydrophobic benefit agent.

### SUMMARY

A method of increasing skin chroma, the method comprising improving skin hydration by applying a multiphase personal cleansing composition comprising a benefit agent to the skin, wherein the level of hydration is in accordance with at least one of the following: a Corneometer improvement of 0.3 Corneometer units or more as compared to a water control after treatment of at least once a day for at least 4 days when measured at 24 hours after the last treatment in accordance with the Corneometer Testing Method; a dry skin grade improvement of about 0.4 units or more at 3 hours after the last treatment of at least once a day for at least 3 days versus the water control when measured in accordance with the Dry Skin Grade Method; or a combination thereof.

### BRIEF DESCRIPTION OF THE DRAWINGS

FIG. 1 illustrates a graphical depiction of a relationship between skin chroma values and corneometer readings;
FIG. 2 illustrates a graphical depiction of a relationship between skin chroma values and dry skin grades;
FIG. 3 illustrates a graphical depiction of a correlation between skin chroma values determined by *in vivo* and *in vitro* methods; and
FIG. 4 illustrates a graphical depiction of a relationship between skin chroma values and the total surface energy of a hydrophobic benefit agent.

### DETAILED DESCRIPTION

This application claims priority from U.S. Provisional App. No. 61/635,886 filed April 20, 2012. While the specification concludes with the claims particularly pointing and distinctly claiming the invention, it is believed that the present invention will be better understood from the following description.

The devices, apparatuses, methods, components, and/or compositions of the present invention can include, consist essentially of, or consist of, the components of the present invention as well as other ingredients described herein. As used herein, "consisting essentially of" means that the devices, apparatuses, methods, components, and/or compositions may include additional ingredients, but only if the additional ingredients do not materially alter the basic and novel characteristics of the claimed devices, apparatuses, methods, components, and/or compositions.

All measurements used herein are in metric units unless otherwise specified.

### I. Definitions

As used herein, the following terms shall have the meaning specified thereafter:
"Anhydrous" refers to those compositions, and components thereof, which are substantially free of water.

"Associative polymer" refers to a water-dispersible polymer comprising hydrophobic groups at an end or pendants to a hydrophilic backbone.

"Chroma" refers to a parameter that measures skin color saturation. A color having a high chroma is vivid, almost completely free of white, while a color having a low chroma is dull, washed-out, and pale in appearance.

"Dry skin" refers to a term used by consumers, cosmetic scientists, and dermatologists. Dry skin can be characterized by a rough, scaly, and/or flaky skin surface, especially in low humidity conditions and is often associated with the somatory sensations of tightness, itch, and pain.

"Multiphase" refers to compositions comprising at least two phases which can be chemically distinct (e.g., a cleansing phase and a benefit phase). Such phases can be in direct physical contact with one another. For example, a personal cleansing composition can be a multiphase personal cleansing composition where phases of the personal cleansing composition can be blended or mixed to a significant degree, but still be physically distinct. In these situations, the physical distinctiveness is undetectable to the naked eye. Likewise, the personal cleansing composition can be a multiphase personal cleansing composition where phases of the personal cleansing composition can be made to occupy separate but distinct physical spaces inside a package. In such a case, the phases can be stored such that they are not in direct contact with one another. In another example, the personal cleansing composition can be a multiphase personal cleansing composition where the phases are in physical contact (i.e., the phases are not separated by a barrier and the phases are not emulsified or mixed to any significant degree) and are visually distinct. Visually distinct phases can take many forms (e.g., phases can appear as striped, marbled).

"Non-associative polymer" refers to a water-dispersible polymer with a relatively uniform hydrophilic backbone lacking hydrophobic groups.

"Non-diseased skin" refers to skin that is generally free of disease, infection, and/or fungus. As used herein, dry skin is considered to be included in non-diseased skin.

"Personal cleansing composition" refers to compositions intended for topical application to skin or hair. Personal cleansing compositions can be rinse-off formulations, in which the product can be applied topically to the skin or hair and then subsequently rinsed within seconds to minutes from the skin or hair with water. The product could also be wiped off using a substrate. The personal cleansing compositions can also be used as shaving aids. The personal cleansing compositions can be in the form of, for example, a liquid, semi-liquid cream, lotion, or gel. Examples of personal cleansing compositions can include but are not limited to bar soap, shampoo, conditioning shampoo, body wash, moisturizing body wash, shower gels, skin cleansers, cleansing milks, hair and body wash, in shower body moisturizer, pet shampoo, shaving preparations, and cleansing compositions used in conjunction with a disposable cleansing cloth.

"Skin" refers to human skin or a synthetic skin mimic, wherein the synthetic skin mimic can be textured on one side with a pattern that resembles the texture and surface energy of human skin and can be used for *in vitro* methods of testing. In particular, the textured side of the skin mimic can be coated with 1, 1, 1-trimethyl-1-pentene that is plasma deposited. The skin mimic can be comprised of a molded bicomponent polyurethane substrate. Suitable skin mimic surface materials are described in co-pending and co-assigned Coated Substrate with Properties of Keratinous Tissue, U.S Patent Pub. No. 20070128255A1 (filed Aug. 11, 2006) (published June 7, 2007) and Methods of Use of Substrate Having Properties of Keratinous Tissue, U.S Patent Pub. No. 20070288186A1 (filed Feb. 5, 2007) (published Dec. 13, 2007).

"Structured" refers to having a rheology that can confer stability on the personal cleansing composition. A degree of structure can be determined by characteristics determined by a Zero Shear Viscosity Method. Accordingly, a structured cleansing phase of the personal cleansing composition can be considered to be structured if the structured cleansing phase has a Zero Shear Viscosity of about 20 Pascal-seconds (Pa-s) or more, about 200 Pa-s or more, about 500 Pa-s or more, about 1,000 Pa-s or more, about 1,500 Pa-s or more, or about 2,000 Pa-s or more. This and other methods for determining characteristics which can define a degree of structure are described in U.S. Patent Published App. No. 2012/0009285.

The phrase "substantially free of" as used herein, unless otherwise specified means that the personal cleansing composition comprises less than about 5%, less than about 3%, less than about 1%, or even less than about 0.1% of the stated ingredient. The term "free of" as used herein means that the personal cleansing composition comprises 0% of the stated ingredient that is the ingredient has not been added to the personal cleansing composition. However, these ingredients may incidentally form as a byproduct or a reaction product of the other components of the personal cleansing composition.

"Visually distinct" generally refers to a region of the multiphase personal cleansing composition having one average composition, as distinct from another region having a different average composition, wherein the regions can be visible to the unaided naked eye. This would not preclude distinct regions from comprising two similar multiphase personal cleansing compositions or phases where one multiphase personal cleansing composition or phase can comprise certain pigments, dyes, particles, and various optional ingredients, hence providing a region of different average composition (e.g., different textures or different colors).

### II. Methods of Increasing Skin Chroma

Healthy skin appearance is a highly desired benefit that consumers seek from soaps, body washes, and other personal cleansing compositions. Such healthy skin can be typically identified by radiant skin possessing a natural color with a high chroma. Deep wrinkles and dry skin flakes, which can result from dehydrated epidermis, can produce regions with high contrast to skin surface, block natural color, and scatter light in different directions generating white spots, thereby negatively affecting such properties associated with a healthy skin appearance.

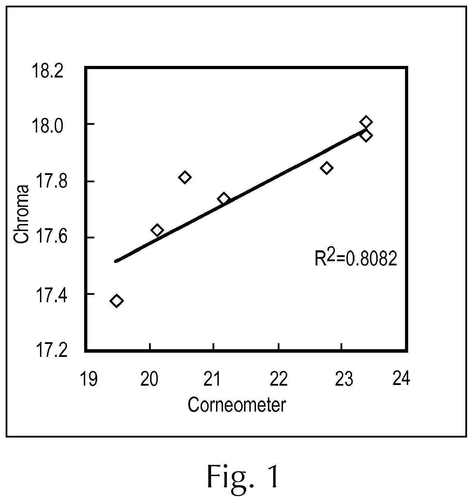

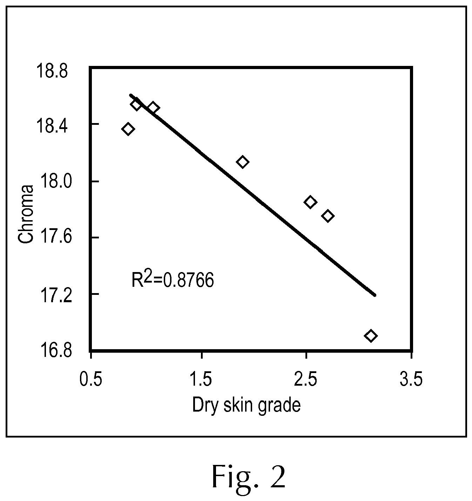

Without wishing to be bound by theory, it is believed that there exists a correlation between healthy skin appearance and overall skin health. In particular, it is believed that skin chroma, an appearance benefit, can be increased by enhancing skin hydration. It is further believed that skin chroma can be increased by decreasing dry skin grade. As shown in FIGS. 1 and 2, as skin chroma increases, skin hydration levels also increase. Specifically, in FIG. 1, as corneometer readings increase (a signal that skin hydration is increasing), the skin also illustrates an increase in chroma values. As depicted in FIG. 2, as dry skin grade values decrease, the skin illustrates an increase in chroma values. As skin chroma can be an indication of healthy skin appearance, an increase in chroma can correspond to a more natural, vivid skin color as well as an improved skin appearance. Data relating to FIGS. 1-2 can be found below in Table 2.

Table 2, below, shows an increase in chroma for skin treated with various personal cleansing compositions relative to no chroma increase for skin treated with a water-based control sample (e.g., Comparative Example 1). Each of Examples 2-7 also have different hydrophobic benefit agents. In particular, the examples illustrated in Table 2 have chroma measurements taken about 3 hours after a last cleansing treatment of a personal cleansing composition, wherein about 5 µg/cm² or more of a hydrophobic benefit agent was deposited on the skin, and wherein a starting dry skin grade of the individual was about 2.5 or more. The chroma of the skin can increase, for example, by about 0.3 units or more; by about 0.5 units or more; by about 0.7 units or more; by about 0.8 units or more; by about 0.9 units or more; by about 1.0 units or more; by about 1.1 units or more; by about 1.5 units or more; or by about 2.0 units or more, after a last cleansing treatment.

To increase skin chroma, a personal cleansing composition having a hydrophobic benefit agent can be applied to the skin. A cleansing treatment, or an application, can include, for example, dispensing about 0.7 mL of a personal cleansing composition onto a center of a treatment area, gently spreading the personal cleansing composition on the treatment area with a wet puff for about 10 seconds, allowing lather to remain on the treatment area for about 90 seconds, rinsing the treatment area for about 15 seconds, taking care not to rinse adjacent areas, and then gently patting the treatment area dry. In other examples, the composition can be applied to the skin for about 60 seconds or more; about 90 seconds or more; or about 120 seconds or more.

An application or treatment can be performed once or can be performed once per day for possibly several days. For example, an application of a personal cleansing composition having a hydrophobic benefit agent can be performed at least once per day for about 2 days or more; for about 3 days or more; for about 5 days or more; or for about 21 days or more.

An increase in chroma can be measurable, for example, at about 3 hours after a last cleansing treatment of the personal cleansing composition or at about 24 hours after application of a last cleansing treatment of the personal cleansing composition. The method for measuring chroma is described below.

A corneometer, which can measure moisture level, can be used to measure improvements in skin hydration. For example, typical Corneometer Units range from about 15-20, wherein the higher the value the higher the level of hydration; and the lower the value, the lower the level of hydration. As described herein, and as shown in FIG. 1, skin chroma can increase with increasing corneometer readings. As such, without being bound by theory, having an increase in chroma of the skin unexpectedly indicates that skin has an increase in hydration. This correlation is illustrated by the graphical data provided in FIG. 1. To further evaluate this correlation, data can be entered into a spreadsheet program (e.g., Microsoft ® Excel ®) to generate a correlation coefficient, R². The closer R² is to 1.00, the closer a correlation is to a mathematical relationship and R² value of about 0.50 or more is typically considered to be acceptable for *in vivo* testing relating to personal cleansing compositions. As shown in FIG. 1, the R² values of 0.8082 indicate the relative level of correlation between the skin chroma measurements and the corneometer readings. As set forth above, data relating to FIG. 1 can be found below in Table 2.

Skin hydration can improve by about 0.2 Corneometer Units or more; by about 0.25 Corneometer Units or more; by about 0.3 Corneometer Units or more; by about 0.4 Corneometer Units or more; by about 0.5 Corneometer Units or more; by about 0.7 Corneometer Units or more; by about 1.0 Corneometer Unit or more, after a last cleansing treatment with a personal cleansing composition described herein. Methods for using a corneometer are described below.

Another method to determine skin hydration levels involves a dry skin grade test. For example, dry skin grades range from about 0.0-6.0, wherein 0.0 corresponds to perfect skin and a high degree of skin hydration and 6.0 refers to severely dry skin having extreme redness and a low degree of hydration. As set forth above, and as illustrated in FIG. 2, skin chroma can increase with decreasing dry skin grades. The graph of FIG. 2 illustrates a R² value of 0.8766 which indicates the level of correlation between the skin chroma measurements and the dry skin grades. As set forth above, data relating to FIG. 2 can be found below in Table 2. Similar to the relationship between the skin chroma measurements and the corneometer testing, the correlation between increasing skin chroma measurements and decreasing dry skin grade values show that a skin chroma increase can correspond to an increase in a level of skin hydration. A method of increasing skin chroma can include decreasing a dry skin grade by about 0.3 units or more; by about 0.35 units or more; by about 0.4 units or more; or by about 0.5 units or more at about 3 hours after a last cleansing treatment of a personal cleansing composition having a hydrophobic benefit agent. The dry skin grade test is described below.

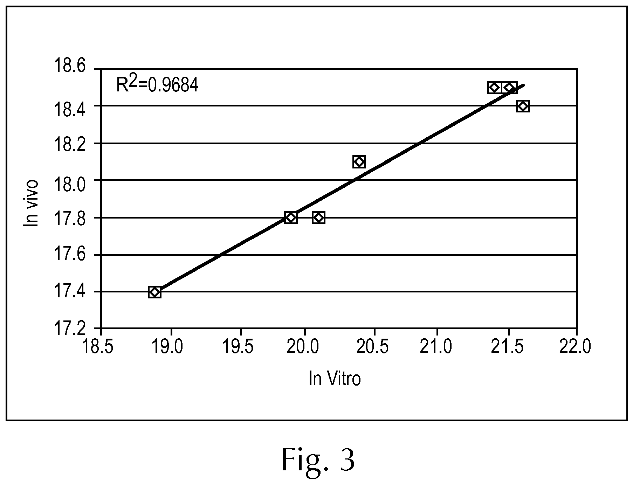

Table 2 shows results from *in vivo* measurements; however, it is believed that *in vitro* methods for skin color enhancement can be used to predict *in vivo* method results in order to build technical models and develop new compositions. Such methodology includes having an *in vitro* deposition evaluation performed along with the chroma test method, both of which are described below. In order to imitate a skin tone, a tan-colored background, for example, can be used underneath a skin mimic when measuring chroma. FIG. 3 illustrates a strong correlation between *in vivo* and *in vitro* measurements, resulting in a R² value of about 0.93. Data relating to FIG. 3 can be found below in Table 2.

Further, chroma measurements from *in vitro* methods can also correlate with several desired consumer attributes relating to skin appearance. For example, upon using personal cleansing compositions, consumers can identify how each composition can affect desired skin-appearance attributes, such as "more radiant," "brighter skin," "look younger," and "visibly healthy" and non-appearance attributes, such as "feel beautiful." Compositions providing such desired skin-appearance attributes, as determined by the consumer testing, can result in high correlations (R² ≥ 0.50) with compositions providing increased chroma measurements.

In the methods for increasing skin chroma, a personal cleansing article can be used to apply the personal cleansing composition. Non-limiting examples of such articles include a sponge or sponge-tipped applicator, a mesh shower puff, a swab, a brush, a wipe (e.g., wash cloth), a loofah, and combinations thereof. In particular, the cleansing article (e.g., faux bar) can be used with the above *in vitro* methods as the cleansing article protocol can achieve results closer to *in vivo* methods described above. Suitable faux bar cleansing articles are disclosed in U.S. Provisional Patent Application Serial No. 61/523,824.

### III. Personal Cleansing Compositions

The method of increasing skin chroma can include depositing a benefit agent on the skin via a personal cleansing composition. The personal cleansing composition can be a multiphase composition. Such multiphase compositions can include at least two phases which can be chemically distinct. For example, personal cleansing compositions can include a cleansing phase and a benefit phase. The benefit phase can include one or more benefit agents that can be deposited on the skin to provide increased skin chroma, enhanced skin hydration, and other desired benefits. As suggested in the literature, there are several benefit agents that can provide such desired benefits. However, providing sufficient deposition of such benefit agents and achieving a desired level of skin hydration can be a difficult task. As a result, there is a continued interest in improving deposition of benefit agents and enhancing skin chroma and hydration.

Without wishing to be bound by theory, it is believed that deposition of a hydrophobic benefit agent can result in increased skin chroma and/or enhanced skin hydration to provide reduced dry skin grade. In particular, it is believed that delivering a hydrophobic benefit agent via a personal cleansing composition to form a more visco-elastic benefit phase and larger particles, which are conducive for the increased deposition that can lead to increased chroma. Such an increase in skin chroma is illustrated by the examples in Tables 3 and 4, provided herein.

For example, Table 3 shows an increase in skin chroma for compositions having soy oligomer with soybean oil as a hydrophobic benefit agent relative to compositions having only soybean oil as a hydrophobic benefit agent. In such compositions having soy oligomer with soybean oil as a hydrophobic benefit agent, the chroma level appears to increase with the percentage of soy oligomer used. Further, Table 4 shows an increase in skin chroma for compositions having soy oligomer with SEFOSE® 1618S as a hydrophobic benefit agent relative to compositions having only SEFOSE® 1618S as a hydrophobic benefit agent.

### A. Cleansing Phase

As noted herein, a personal cleansing composition can include a cleansing phase and a benefit phase. The cleansing phase can comprise one or more surfactants. Such surfactants can be suitable for application to skin and hair and are compatible with other components of the cleansing phase, including water. A personal cleansing composition comprising the cleansing phase, can comprise, for example, from about 1% to about 30%, from about 5% to about 25%, or from about 15% to about 22%, by weight of the personal cleansing composition, of surfactants.

The surfactants can include anionic (e.g. sodium laureth sulfate, ammonium lauryl sulfate and sodium trideceth sulfate), nonionic (e.g. isosteareth-2, trideceth-3, TDA-3), cationic, zwitterionic (e.g. cocoamidopropyl betaine), amphoteric surfactants (e.g. sodium lauroamphoacetate, sodium cocoamphoactetate and disodium lauroamphoacetate) and mixtures thereof. Suitable surfactants for the multiphase personal care compositions are described in McCutcheon's: Detergents and Emulsifiers North American Edition (Allured Publishing Corporation 1947) (1986), McCutcheon's, Functional Materials North American Edition (Allured Publishing Corporation 1973) (1992) and U.S. Pat. No. 3,929,678 (filed Aug. 1, 1974).

The cleansing phase can be a structured cleansing phase or, as shown in Table 5, the cleansing phase can be a non-structured cleansing phase. In a structured cleansing phase, the structured cleansing phase can include various surfactant chassis, such that the structured cleansing phase includes at least one surfactant (e.g., STnS). A structured cleansing phase can include, for example, from about 1% to about 20%, from about 2% to about 15%, from about 5% to about 20%, or from about 5% to about 20%, by weight of the personal cleansing composition of STnS, wherein n can define average moles of ethoxylation. n can range from about 0 to about 3; from about 0.5 to about 2.7; from about 1.1 to about 2.5; from about 1.8 to about 2.2, or n can be about 2. When n can be less than 3, STnS can provide improved stability, improved compatibility of benefit agents within the personal cleansing compositions, and increased mildness of the personal cleansing compositions, such described benefits of STnS are disclosed in U.S. Patent Application Serial No. 13/157,665.

The surfactant can also comprise one or more branched anionic surfactants and monomethyl branched anionic surfactants such as sodium tridecyl sulfate, sodium C₁₂-C₁₃ alkyl sulfate, and C₁₂-C₁₃ pareth sulfate and sodium C₁₂-C₁₃ pareth-n sulfate.

The cleansing phase can comprise at least one anionic surfactant, such as sodium lauryl sulfate ("SLS"). Suitable examples of SLS are described in U.S. Patent Application Serial No. 12/817,786. Another suitable surfactant, can include sodium laureth(n) sulfate, hereinafter SLEnS, wherein n can define average moles of ethoxylation. n can range from about 1 to about 3. Other suitable anionic surfactants can include ammonium lauryl sulfate, ammonium laureth sulfate, potassium laureth sulfate, sodium lauryl sarcosinate, sodium lauroyl sarcosinate, lauryl sarcosine, cocoyl sarcosine, ammonium cocoyl sulfate, potassium lauryl sulfate, and combinations thereof. Alternatively, the personal cleansing composition can be optionally free of SLS and can comprise at least a 70% lamellar structure.

As described above, the surfactant can include nonionic and cationic surfactants. Nonionic surfactants for use in the personal cleansing composition can include those selected from the group consisting of alkyl glucosides, alkyl polyglucosides, polyhydroxy fatty acid amides, alkoxylated fatty acid esters, sucrose esters, amine oxides, and mixtures thereof. Cationic surfactants for use in the personal cleansing composition can include, but are not limited to, fatty amines, di-fatty quaternary amines, tri-fatty quaternary amines, imidazolinium quaternary amines, and combinations thereof.

Suitable amphoteric surfactants can include those that can be broadly described as derivatives of aliphatic secondary and tertiary amines in which an aliphatic radical can be straight or branched chain and wherein an aliphatic substituent can contain from about 8 to about 18 carbon atoms such that one carbon atom can contain an anionic water solubilizing group, e.g., carboxy, sulfonate, sulfate, phosphate, or phosphonate. Examples of compounds falling within this definition can be sodium 3-dodecyl-aminopropionate, sodium 3-dodecylaminopropane sulfonate, sodium lauryl sarcosinate, N-alkyltaurines such as the one prepared by reacting dodecylamine with sodium isethionate according to the teaching of U.S. Pat. No. 2,658,072, N-higher alkyl aspartic acids such as those produced according to the teaching of U.S. Pat. No. 2,438,091, and products described in U.S. Pat. No. 2,528,378. Other examples of amphoteric surfactants can include sodium lauroamphoacetate, sodium cocoamphoactetate, disodium lauroamphoacetate disodium cocodiamphoacetate, and mixtures thereof. Amphoacetates and diamphoacetates can also be used.

Zwitterionic surfactants suitable for use as described above can include those that are broadly described as derivatives of aliphatic quaternary ammonium, phosphonium, and sulfonium compounds, in which aliphatic radicals can be straight or branched chains, and wherein an aliphatic substituent can contain from about 8 to about 18 carbon atoms such that one carbon atom can contain an anionic group, e.g., carboxy, sulfonate, sulfate, phosphate, or phosphonate. Other zwitterionic surfactants can include betaines, including cocoamidopropyl betaine. Additional suitable amphoteric or zwitterionic surfactants can include those described in U.S. Patent Nos. 5,104,646 and 5,106,609.

A cleansing phase can also include an associative and/or non-associative polymer. Associative polymers used in the cleansing phase can be a crosslinked, alkali swellable, associative polymer comprising acidic monomers and associative monomers with hydrophobic end groups, whereby the associative polymer comprises a percentage hydrophobic modification and a hydrophobic side chain comprising alkyl functional groups. Without intending to be limited by theory, it is believed the acidic monomers can contribute to an ability of the associative polymer to swell in water upon neutralization of acidic groups; and associative monomers anchor the associative polymer into structured surfactant hydrophobic domains, e.g., lamellae, to confer structure to the surfactant phase and keep the associative polymer from collapsing and losing effectiveness in a presence of an electrolyte. Associative alkyl side chains can comprise, for example, butyl, propyl, stearyl, steareth, cetyl, lauryl, laureth, octyl, behenyl, beheneth, steareth, or other linear, branched, saturated, or unsaturated alkyl or alketh hydrocarbon side chains.

A suitable associative polymer is AQUPEC® SER-300 made by Sumitomo Seika of Japan, which is an acrylate/C₁₀-C₃₀ alkyl acrylate cross-polymer and comprises stearyl side chains with less than about 1% HM (hydrophobic modification). Associative polymers can comprise about C₁₆ (cetyl) alkyl hydrophobic side chains with about 0.7% hydrophobic modification, but a percentage hydrophobic modification can be up to an aqueous solubility limit in surfactant compositions (e.g., up to 2%, 5%, or 10%). Other associative polymers can include stearyl, octyl, decyl and lauryl side chains, alkyl acrylate polymers, polyacrylates, hydrophobically-modified polysaccharides, hydrophobically-modified urethanes, AQUPEC® SER-150 (acrylate/C₁₀-C₃₀ alkyl acrylate cross-polymer) comprising about C₁₈ (stearyl) side chains and about 0.4% HM, and AQUPEC® HV-701EDR which comprises about C₈ (octyl) side chains and about 3.5% HM, and mixtures thereof. One exemplary associative polymer can be Stabylen 30 manufactured by 3V Sigma S.p.A., which has branched isodecanoate hydrophobic associative side chains.

The cleansing phase of a personal cleansing composition can further include a non-associative polymer. Suitable non-associative polymers can include water-dispersible polymers with relatively uniform hydrophilic backbone lacking hydrophobic groups. Examples of non-associative polymers can include biopolymer polysaccharides (e.g., xanthan gum, gellan gum), cellulosic polysaccharides (e.g., carboxymethyl cellulose, carboxymethyl hydroxyethyl cellulose), other polysaccharides (e.g., guar gum, hydroxypropyl guar, and sodium alginate), and synthetic hydrocarbon polymers (e.g., polyacrylamide and copolymers, polyethylene oxide, polyacrylic acid copolymers).

Personal cleansing compositions can additionally comprise an organic cationic deposition polymer in one or more phases as a deposition aid for benefit agents described herein. Suitable cationic deposition polymers can contain cationic nitrogen-containing moieties such as quaternary moieties. Non-limiting examples of cationic deposition polymers can include polysaccharide polymers, such as cationic cellulose derivatives. Cationic cellulose polymers can be salts of hydroxyethyl cellulose reacted with trimethyl ammonium substituted epoxide, referred to in the industry (CTFA) as Polyquaternium 10, which can be available from Amerchol Corp. (Edison, N.J.) in their Polymer KG, JR, and LR series of polymers. Other suitable cationic deposition polymers can include cationic guar gum derivatives, such as guar hydroxypropyltrimonium chloride, specific examples of which can include the Jaguar series commercially available from Rhodia Inc. and N-Hance polymer series commercially available from Aqualon. Suitable water-soluble cationic deposition polymers can include synthetic polyacrylamides such as Polyquaternium 76 and Polymethylene-bis-acrylamide methacrylamido propyltrimethyl ammonium chloride (PAM/MAPTAC). Such PAM/MAPTAC can have an acrylamide to methacrylamido propyltrimethyl ammonium chloride ratio of 88:12. The deposition polymers can have a cationic charge density from about 0.8 meq/g to about 2.0 meq/g; or from about 1.0 meq/g to about 1.5 meq/g.

A cleansing phase of a personal cleansing composition can also include water. The cleansing phase can comprise, for example, from about 10% to about 90%, from about 40% to about 85%, or from about 60% to about 80%, by weight of the cleansing phase, of water.

Other optional additives can be included in the cleaning phase, including for example an emulsifier (e.g., non-ionic emulsifier) and electrolyes. Suitable electrolytes can includes an anion such as phosphate, chloride, sulfate, citrate, and mixtures thereof and a cation such as sodium, ammonium, potassium, magnesium, and mixtures thereof. For example, suitable electrolytes can include sodium chloride, ammonium chloride, sodium sulfate, ammonium sulfate, and mixtures thereof. Other suitable emulsifiers and electrolytes are described in U.S. Patent Application Serial No. 13/157,665.

### B. Benefit Phase

As noted herein, personal cleansing compositions can include a benefit phase. The benefit phase can be hydrophobic and/or anhydrous. The benefit phase can also be substantially free of any surfactant.

The benefit phase can include one or more benefit agents. For example, the benefit phase can comprise from about 0.1% to about 50%, from about 0.5% to about 20%, or from about 1.0% to about 10%, by weight of the personal cleansing composition, of the benefit agent. Such benefit agents can include hydrophobic benefit agents.

Non-limiting examples of benefit agents can include petrolatum, glyceryl monooleate, mineral oil, natural oils (e.g., soybean oil, saturated or unsaturated), sucrose esters, cholesterol, fatty esters, fatty alcohols, petrolatum, glyceryl monooleate, zinc phrithione, Olivem 1000, and mixtures thereof. Other suitable benefit agents are described in U.S. Patent Application Serial No. 13/157,665.

Additional non-limiting examples of benefit agents include SEFOSE®, lanolin esters, lanolin oil, natural waxes, synthetic waxes, volatile organosiloxanes, derivatives of volatile organosiloxanes, non-volatile organosiloxanes, derivatives of non-volatile organosiloxanes, natural triglycerides, synthetic triglycerides, polyglycerides, and combinations thereof.

Another suitable benefit agent comprises a metathesized unsaturated polyol ester. A metathesized unsaturated polyol ester refers to the product obtained when one or more unsaturated polyol ester ingredient(s) are subjected to a metathesis reaction. Metathesis is a catalytic reaction that involves the interchange of alkylidene units among compounds containing one or more double bonds (i.e., olefinic compounds) via the formation and cleavage of the carbon-carbon double bonds. When the unsaturated poyol ester comprises molecules that have more than one carbon-carbon double bond (i.e., a polyunsaturated polyol ester), self-metathesis results in oligomerization of the unsaturated polyol ester.

As a starting material, metathesized unsaturated polyol esters are prepared from one or more unsaturated polyol esters. Exemplary unsaturated polyol esters are described in detail in U.S. 2009/0220443 A1. Additionally, suitable unsaturated polyol esters can include an unsaturated ester of glycerol. Sources of unsaturated polyol esters of glycerol include synthesized oils, natural oils (e.g., vegetable oils, algae oils, bacterial derived oils, and animal fats), combinations of theses, and the like. Recycled used vegetable oils may also be used. Representative examples of vegetable oils include argan oil, canola oil, rapeseed oil, coconut oil, corn oil, cottonseed oil, olive oil, palm oil, peanut oil, safflower oil, sesame oil, soy-bean oil, sunflower oil, high oleoyl soy-bean oil, high oleoyl sunflower oil, linseed oil, palm kernel oil, tung oil, castor oil, high oloeyl sunflower oil, high oleoyl soybean oil, high erucic rape oils, Jatropha oil, combinations of theses, and the like. Representative examples of animal fats include lard, tallow, chicken fat, yellow grease, fish oil, combinations of these, and the like. A representative example of a synthesized oil includes tall oil, which is a byproduct of wood pulp manufacture.

Other examples of unsaturated polyol esters include diesters such as those derived from ethylene glycol or propylene glycol, esters such as those derived from pentaerythritol or dipentaerythritol, or sugar esters such as SEFOSE®. Sugar esters such as SEFOSE® include one or more types of sucrose polyesters, with up to eight ester groups that could undergo a metathesis exchange reaction.

Non-limiting examples of sucrose polyesters suitable for use include sucrose polysoyate (SEFOSE® 1618S), SEFOSE® 1618U, SEFOSE® 1618H, Sefa Soyate IMF 40, Sefa Soyate LP426, SEFOSE® 2275, SEFOSE® C1695, SEFOSE® C18:0 95, SEFOSE® C1495, SEFOSE® 1618H B6, SEFOSE® 1618S B6, SEFOSE® 1618U B6, sucrose polycottonseedate, SEFOSE® C1295, Sefa C895, Sefa C1095, SEFOSE® 1618S B4.5, all available from The Procter and Gamble Co. of Cincinnati, Ohio.

Non-limiting examples of glycerides suitable for use as hydrophobic skin benefit agents herein can include castor oil, safflower oil, corn oil, walnut oil, peanut oil, olive oil, cod liver oil, almond oil, avocado oil, palm oil, sesame oil, soybean oil, unsaturated soybean oil, vegetable oils, sunflower seed oil, vegetable oil derivatives, coconut oil and derivatized coconut oil, cottonseed oil and derivatized cottonseed oil, jojoba oil, cocoa butter, and combinations thereof.

Non-limiting examples of silicone oils suitable for use as hydrophobic skin benefit agents herein can include dimethicone copolyol, dimethylpolysiloxane, diethylpolysiloxane, mixed C₁-C₃₀ alkyl polysiloxanes, phenyl dimethicone, dimethiconol, and combinations thereof. Non-limiting examples of silicone oils useful herein are described in U.S. Patent No. 5,011,681. Still other suitable hydrophobic skin benefit agents can include milk triglycerides (e.g., hydroxylated milk glyceride) and polyol fatty acid polyesters.

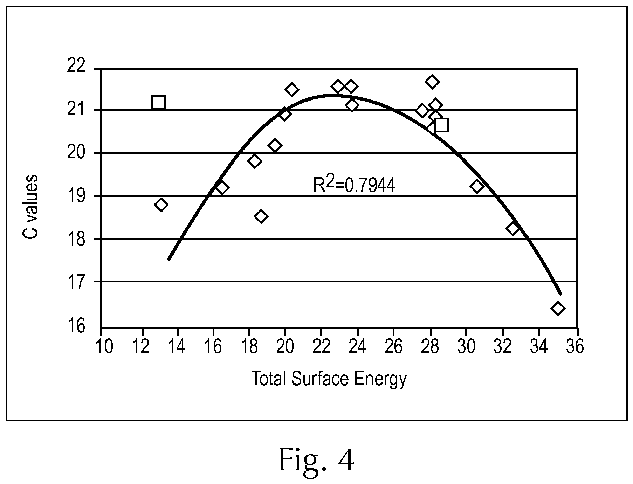

Where the hydrophobic benefit agent can include SEFOSE®, hydrophobic benefit agents can exhibit a total surface energy of about 20 mJ/m² to about 30 mJ/m² and a change in heat of fusion (or Δ heat of fusion) of about 50 J/g or less, which provide for favorable deposition. As shown in FIG. 4, sucrose polyesters having a total surface energy of about 20 mJ/m² to about 30 mJ/m², a range comparable to that of skin in the presence of a surfactant, can be thermodynamically favorable for deposition such that the similar surface energies can promote adhesion and spreading. Further, using a Δ heat of fusion of about 50 J/g or less can ensure that the sucrose polyester can be fluid-like for adequate coverage and spreading. Such properties can be favorable for enhanced chroma measurements because, and as described herein, chroma of the skin can be increased through increased deposition of the hydrophobic benefit agent.

The hydrophobic benefit agents as described herein can be combined with a soy oligomer. The benefit phase can include, for example, from about 1% to about 50%, from about 2% to about 40%, from about 3% to about 30%, from about 4% to about 20%, or from about 5% to about 15%, by weight of the benefit phase, of a soy oligomer. The soy oligomer can be fully or partially hydrogenated. For example the soy oligomer can be about 60% hydrogenated or more; about 70% hydrogenated or more; about 80% hydrogenated or more; about 85% hydrogenated or more; about 90% hydrogenated or more; or 100% hydrogenated. In particular, the soy oligomer can be DOW CORNING® HY-3050 soy wax or DOW CORNING® HY-3051 soy wax blend, both available from Dow Corning.

A hydrophobic benefit agent can exhibit a Vaughan solubility parameter from about 5 to about 14 and exhibit a viscosity of about 1500 cP or less at from about 20°C to about 25°C. Vaughan solubility parameters are defined in Vaughan in Cosmetics and Toiletries, Vol. 103. Non-limiting examples of hydrophobic materials having Vaughan solubility parameter values in the above range can include the following: Cyclomethicone, 5.92; Squalene, 6.03; Petrolatum, 7.33; Isopropyl Palmitate, 7.78; Isopropyl Myristate, 8.02; Castor Oil, 8.90; Cholesterol, 9.55; as reported in Solubility, Effects in Product, Package, Penetration and Preservation, C. D. Vaughan, Cosmetics and Toiletries, Vol. 103, October 1988.

### IV. Test Methods

### A. Skin Preparation Method

Prior to the Chroma Testing, Dry Skin Grade Screening, and Corneometer Testing, subjects undergo the following skin treatment. Prior to a study, subjects participate in a washout period for seven days, in which the subjects only use soap that is provided to them (e.g., soap including shea butter and no beads) and abstain from washing their legs with any other products. Subjects are also instructed to abstain from applying any leave-on products to their legs during the pre-study washout period.

A clinical assistant will mark 2-7 cm (across) x 10 cm (down) treatment sites on an outer portion of the lower legs using a template and a laboratory marking pen (4 corner brackets are sufficient to delineate each area). For assignment of the products, two sites located on the left leg will be numbered L1 and L2, where L1 is the top part of the lower leg nearest the knee, and L2 is the bottom part of the lower leg nearest the ankle. Two sites located on the right leg will be numbered R1 and R2, where R1 is the top part of the lower leg nearest the knee, and R2 is the bottom part of the lower leg nearest the ankle.

To simplify the treatment process, master trays will be prepared for each treatment plan specified in the study randomization. Each master tray will be divided in half, with each half labeled 'left' or 'right' to indicate which leg it corresponds to, then subdivided into sections for the test products in the order of leg application site. One or more make-up trays can also be prepared for use as needed using individual coded containers, or other appropriate product code indicators, that can be re-arranged according to a given treatment plan.

Trained clinical assistants will wash each subject's lower legs in a controlled manner with assigned treatments once daily for 21 consecutive days. Assignment of test treatments to skin sites on the left and right legs will be designated by study randomization. A target dose of body wash for each site is 10 µL/cm². All body wash products will be dispensed at 0.7 mL dosages. All body wash test products will be drawn up into syringes at the 0.7 mL dosage. A one day supply of syringes for all products may be filled the day before or the day of use. Product that has been transferred to another container and the container itself will be used for one day only (i.e., the day the transfer occurred). All syringe filling operations will be appropriately documented (e.g., product code filled, when filled, initials of person responsible for filling).

The treatment area on the top part of the left leg of the subject is wetted for 5 seconds with 95-100°F (35 to 37.8°C) running tap water. The water flow rate is about 1200 mL per minute. For the "No Treatment" site, apply water only. For a treatment site, dispense 0.7 mL of body wash product from the syringe onto the center of the treatment area and place a wet puff over the dispensed product and gently rub the puff back and forth within the treatment site for 10 seconds. Then, allow lather (or water only) to remain on the site for 90 seconds. When residence time for a site has expired, the site is rinsed for 15 seconds under a running tap, taking care not to rinse adjacent sites. After the application area has been rinsed, the area is gently patted dry. Repeat the procedure for the lower part of the left leg, and after completion, use the same procedure for each of the top part of the right leg and the lower part of the right leg.

### B. Chroma Test Method

To measure skin chroma, a MicroFlash spectrometer D200 manufactured by Datacolor can be used. First turn on the MicroFlash and the computer, opening Datacolor Tools software. Log in to arrive at the measurement screen. Using the MicroFlash Instructions, calibrate the instrument such that the calibration is done in specular "Include" mode. Click "Instruments" in the upper toolbar and choose "Calibrate." Once the "Set Black Trap" window appears, place the MicroFlash head over the black trap and click "Ready." Once the "Set White Tile" window appears, place the MicroFlash head over the white tile and click "Ready." Then, once the "Set Up Instrument for Diagnostic Tile Measurement" window appears, place the MicroFlash head on the green tile and click "Ready." "Pass" should be indicated in the lower portion of the window. At this point, note ΔE, which should be less than 0.2. Go to "List Standard" and click "Green Tile Included." Enter a batch name, and once again, measure the green tile. ΔE should be less than 0.2 and should be similar to ΔE value previously shown on the green tile calibration screen.

Measurements should be taken in specular "Excluded" mode. Click on "New Standard" and enter a new standard name. Place the MicroFlash head against a flat surface and squeeze the trigger to save a new standard name, and then type in a new Batch Name. Place the MicroFlash head on a sample and squeeze the trigger to collect data.

Before measuring skin chroma via the *in-vivo* method, skin is prepared using the Skin Preparation Method described above. Skin color measurements are taken for each of areas L1, L2, R1, and R2, and measurements can be recorded for several time increments. Such measurements are taken with three replicates at each time point for each area, where each replicate is spread out evenly on the diagonal line of the area. For example, measurements can be recorded at the beginning of testing (as a baseline), 3 hours after a first cleansing treatment, 24 after the first cleansing treatment, 24 hours after a fourth treatment, 3 hours after a fifth treatment, 24 hours after an eleventh treatment, and 3 hours after a twelfth treatment. Such measurements are used, for example, in Table 2 and Figures 1-3.

To measure skin chroma for the *in-vitro* method, use the Fastmice skin mimic sheets as substrate, along with the 12-well plates, to generate 6 replicates of each product divided between two plates using a 1:29 dilution. The Fastmice method includes a 5-minute application period and a water-only set (average water chroma is about 21.0 ± 1.0) on each plate as a control. Upon completing the Fastmice samples, place the skin mimic atop a tan folder (Smead, SuperTab Folders, Assorted Pastels, No. 11961 - tan is one of the colors included in this set, and the average folder chroma is 29.5 ± 1.0). Take three readings of the folder, as a check. Because the folders fade over time, a check needs to be done before each run to validate that the chroma of the folder itself has not undergone extreme variation.

Using the MicroFlash Instruction sheets, read each sample of the six replicates, generating color readings for each sample. Pulling the generated chroma data only, average the six readings for each product, and then chart and graph as needed.

### C. Dry Skin Grade Screen

Test subjects are screened for dry skin grade of 2.5-4.0 by trained expert graders following guidelines below. Preparation for dry skin grade screening is the same as that used prior to the *in-vivo* chroma test method described above. As set forth above, before initial visual grading, subjects must undergo the Skin Preparation Method referenced above. Visual evaluations will be done with the aid of an Illuminated Magnifying Lamp which provides 2.75X magnification and which has a shadow-free circular fluorescent light source (General Electric Cool White, 22 watt 8" Circline). At least 30 subjects are needed to obtain sufficient replicates for each treatment. Table 1 shows a grading scale for dry skin and lists the redness and dryness characteristics associated with each grade.

**Table 1.**

| Grade* | Redness | Dryness** |
|---|---|---|
| 0.0 | No redness | Perfect skin |
| 1.0 | Barely detectable redness | Patches of checking and/or slight powderiness, occasional patches of small scales may be seen, distribution generalized |
| 2.0 | Slight redness | Generalized slight powderiness, early cracking, or occasional small lifting scales may be present |
| 3.0 | Moderate redness | Generalized moderate powderiness and/or heavy cracking and lifting scales |
| 4.0 | Heavy or substantial redness | Generalized heavy powderiness and/or heavy cracking and lifting scales |
| 5.0 | Severe redness | Generalized high cracking and lifting scales, eczematous change may be present, but not prominent, may see bleeding cracks |
| 6.0 | Extreme redness | Generalized severe cracking, bleeding cracks and eczematous changes may be present, large scales may be sloughing off |
| *Half-unit grades may be used if necessary | | |
| **"Generalized" refers to situations where more than 50% of an application area is affected | | |

### D. Corneometer Testing

Once the materials are applied as noted above in Section A, the Skin Preparation Method, improvements in skin hydration can be measured with a Corneometer, while baseline measurements are taken prior to application of materials. In particular, skin hydration based upon measurements of capacitance can be assessed using the Corneometer® 825. Such use of a Corneometer is set forth in U.S. Patent Application Serial No. 13/007,630. Such measurements can be non-invasive and can be taken in duplicate on each site of the subjects' legs at the following times: At baseline, prior to 1^{st} treatment; 3 hours post 1^{st}, 3^{rd}, 5^{th} 14^{th} and 21^{st} treatments; 24 hours post 4^{th}, 13^{th} and 21^{st}, treatments, 48 hours post 21^{st} treatment after a visual assessment has been completed. Subjects can be acclimated for a minimum of thirty minutes in an environmentally controlled room (maintained at 70°F ± 2 (21.1°C) and 30-45% relative humidity) prior to the non-invasive instrumental measurements taken on their legs. Data can be recorded electronically using a Sponsor's direct data entry and data capture programs. Measurements can be performed according to a test facility's standard operating procedures and/or the Sponsors Instrument Operation Manual.

The Corneometer values are arbitrary units for electrical impedance. At baseline, for subjects having a dry skin grade from about 2.5 to about 4.0, an adjusted mean of such Corneometer values can typically fall within a range of about 15 to about 20. Higher Corneometer values can correspond to a higher hydration level, and thus, lower Corneometer values can correspond to lower hydration levels.

The instrument should only be operated by trained operators. Further, the same instrument(s) and operator(s) can be used throughout the study. Kimwipes can be used to wipe an end of a probe. The probe can be wiped with a Kimwipe between each measurement. At the end of an evaluation session, data collected for that period can be backed up according to instructions in the Sponsors Instrument Operation Manual, and a hard copy of the data can be printed.

### E. In-vitro Deposition Evaluation Method

The *in-vitro* Deposition Evaluation Method measures the deposition of benefit agents on a skin mimic. The method compares the quantity of benefit agent of the skin mimic surface before and after cleansing in an automated cleansing unit, such as the automated cleansing unit described in co-pending and co-assigned Multiphase Personal Care Composition With Enhanced Deposition, U.S. Application No. 12/510,880 (filed July 28, 2009) and In-Vitro Deposition Evaluation Method for Identifying Personal Care Compositions Which Provide Improved Deposition of Benefit Agents, U.S. Application No. 12/511,034 (filed July 28, 2009).

The *in-vitro* Deposition Evaluation Method uses two 12-well plates (hereinafter referred to as "plates"). Suitable 12-well plates are commercially available from Greiner bio-one. For example, the Cellstar® 12-well suspension culture plate has 3 rows and 4 columns with a well volume of about 6.2 mL. The Cellstar® 12-well suspension culture plate comprises the approximate dimensions of 19 mm in height, 127 mm in length and 85 mm in width. The Cellstar® 12-well suspension culture plate has a well diameter of 23 mm, a well depth of 15 and a well to well spacing of 2 mm. A Cellstar® 12-well suspension culture plate is provided for containing the samples comprising the personal cleansing composition as described in the Examples herein.

The in-vitro Deposition Evaluation Method uses approximately 120 g of bodies for two plates. Five grams of bodies carefully loaded into each of the 12 wells of the two plates to ensure the same quantity is loaded into each well. Each body is a spherical stainless steel bearing that is approximately 2 mm in circumference. Each body comprises ferrometallic material. Suitable bodies are those available from WLB Antriebeselemente Gmbh, Scarrastrasse 12, D-68307 Mannheim, Germany.

The personal cleansing compositions can be prepared as described by the examples herein. After the examples of the personal cleansing compositions are prepared, control and test samples are prepared by determining the dilution ratio and dispensing both the personal cleansing composition and distilled water into the wells of the microplate and allow the samples to mix while being exposed to the automated washing process. The dilution ratio used in this application is one part of composition and twenty nine parts of water (1:29). A pre-calibrated positive displacement pipette is used to dispense 66.7 µL of composition on to the bodies in each well, followed by dispensing 1933.3 µL of distilled water into each well. The control samples and test samples are dispensed in the specified wells of the plate, all within a 20-minute time frame. Each composition is placed in 6 different well, 3 of which are in plate 1 and the other 3 well are in plate 2. A test control composition containing the benefit agent should be used in every test to ensure consistency among tests.

As set forth above, the skin mimic used in the *in-vitro* Deposition Evaluation Method is comprised of a molded bicomponent polyurethane substrate. The skin mimic is textured on one side with a pattern that resembles the texture of human skin. The textured side of the skin mimic is coated with 1, 1, 1-trimethyl-1-pentene that is plasma deposited. The skin mimic surface has a total surface energy of 32 ±1.0 (mJ/m²) and a contact angle in water of 100°±2.0. Suitable skin mimic surface materials are described in co-pending and co-assigned Coated Substrate with Properties of Keratinous Tissue, U.S Patent Pub. No. 20070128255A1 (filed Aug. 11, 2006) (published June 7, 2007) and Methods of Use of Substrate Having Properties of Keratinous Tissue, U.S Patent Pub. No. 20070288186A1 (filed Feb. 5, 2007) (published Dec. 13, 2007).

After all of the wells of the plate are filled with the samples and the pieces of skin are made and coated, the skin mimic is prepared for the *in-vitro* Deposition Evaluation Method. Two pieces of skin mimic are prepared by cutting the skin mimic to fit on top of all 12 openings of the wells of the plate while wearing gloves. The two pieces of skin mimic pieces are numbered "1" and "2."

Next, the pieces of skin mimics are arranged over the openings of the wells of the microplates. The pieces of skin mimic surface material are transferred to cover the openings of the wells of the each of the plates to ensure that the textured and treated region of the skin mimic is facing the openings of the wells of the plate. A lid is placed over each piece of the skin mimic and the associated plate to form a lidded plate.

The lidded plates are placed into plate holders of an automated cleansing unit, or, a device used in the *in-vitro* Deposition Evaluation Method of the present invention. The automated cleansing unit comprises a horizontal base comprising four microplate holders. The horizontal base is made of rectangle of aluminum comprising the following approximate dimensions of 3/8 inch or 0.95 cm in height, fourteen inches or 35.56 cm in width and twenty seven inches or 68.58 cm in length. The automated cleansing unit further comprises two vertical supports comprised of aluminum with the approximate dimensions of one inch or 2.54 cm by two inches or 5.08 cm by ten and 3/4 of an inch or 27.31cm in height. The vertical supports are attached to a horizontal support comprising a rodless air slide. The horizontal support comprising a rodless air slide comprises the approximately dimension of a ½ inch or 1.27 cm two inches or 5.08 cm by twenty six and ½ inches for 67.31 cm in height. Suitable rodless air slides comprise a one inch bore and eleven inch stroke and have associated end lugs and mount brackets, which are commercially available from McMaster-Carr. The rodless air slide can be double acting and comprises a carriage that is connected to an internal piston and two compressed air ports.

The automated cleansing unit comprises two magnetic arms. The horizontal support comprising a rodless air slide is the structure upon which the two magnetic arms are mounted. The magnetic arms are mounted to the rodless air slide such that the magnetic arms move back and forth along the length of the double acting rodless air slide by the force of compressed air. Each of the magnetic arms are comprised of aluminum and have the approximate dimensions of one inch or 2.54cm by two inches or 5.08 cm by fourteen inches or 35.56 cm in length and have a "T" shape channel that houses seven neodymium iron boron magnets (not shown). Each of the neodymium iron boron magnets has the approximate dimensions of two inches or 5.08 cm in length, one inch or 2.54 cm in width and half or an inch (1.27 cm or 2.54 cm) in height. Each of the neodymium iron boron magnets comprises a magnetic strength of 12200 Gauss, available from Edmund Scientifics. The magnetic arms are configured at a height of about 2.75 cm above the microplate holder with the caveat that the magnets maintain their function to attract and move the bodies comprised within the wells of the microplate. The magnetic arms move back and forth along the length of the rodless air slide by the force of compressed air at a speed of approximately 6 back and forth sweeps over the length of the rodless air slide over a 10 second time period.

The magnetic arms can be configured with four microplate holders. Each of the microplate holders comprise a clamping plate and four pistons attached to a pneumatic control unit. When actuated, the pistons for the pneumatic control unit hold the plates in the four plate holders at a pressure of about 90 psi. or 0.621 mPa. Prior to placing the lidded plates into the plate holders of automated cleansing unit, the pneumatic control unit is turned on.

The automated cleansing unit can comprise a pneumatic control unit according. The top view shows components of the pneumatic control unit which can be connected to the rodless air slide, the piston and clamping plates. The pneumatic control unit can be used to apply compressed air to the automated cleansing unit, which imparts a force by converting the potential energy of compressed air into kinetic energy. The pneumatic control unit comprises a solenoid air control valve, a distribution manifold outlet, a compressed air control valve, a compressed air flow regulator, an alternating output binary valve, a two-hand safety pneumatic control valve, a compressed air control valve and various connectors that provide pressurized air to the automated cleansing unit from an external air source. The air control valve, air flow regulators, alternating a binary valves, a two-hand safety pneumatic control valve are positioned upstream of a solenoid air control valve. A suitable solenoid air control valve is described as a double air style valve with a 10 psi to 120 psi operating pressure. Suitable compressed air flow regulators operate in the pressure range of 14 psi to 116 psi. Suitable air control valve alternating output binary valves operate in a 35 psi to 100 psi range. All of the components of the pneumatic control unit are available from McMaster-Carr®.

The lidded plates are placed into the plate holders and pneumatic control unit is actuated such that the lidded plates are held under 90 psi or 0.621 mPa of pressure. The magnetic arms are actuated on and arms moves over the lidded microplates at a height of 2.65 cm above the plate holders. The magnetic arms of the automated cleansing unit, sweep back and forth over the plate holders for 5 minutes, at a speed of 6 sweeps per every 10 seconds. After 5 minutes of the automated cleansing process, the lidded plates are removed from the plate holders and are disassembled.

After the automated washing process, two large 4000 mL beakers of 20°C to 25°C water are filled. The first piece of skin mimic is removed from the first plate and submerged in the tap water within the first beaker five times. The second piece of skin mimic is removed from the second microplate and submerged within the second beaker five times. The completeness of rinsing step is judged visually by the lack of foam on the skin mimic and presence of defined circles of deposited material on the skin mimic. Both piece of skin mimic are blotted gently with paper towels and fumed in a drying hood for at least 3 hours each.

Clean the blades of the 12-well die with alcohol and Q-tips and the clear cutter plate with Dawn & tap water. Dry the clear cutter plate with paper towels. Next, gently place the first piece of skin mimic, deposit side down, onto the 12-well die, lining up the deposit sites with the circle blades. Gently place the clear cutter lid over the first piece of skin mimic, again lining up the circles of the lid with the deposit sites & circle blades below, and then place this whole die unit into the pneumatic cutter. Operate the cutter dual-switch with both hands, holding for a few seconds to ensure a good cut. Remove the die unit, and then gently lift the clear lid up and over to examine the cut mimic pieces.

Position the labeled glass vials nearby to correspond with the position of the mimic on the clear lid, according to rows & columns labeled previously on the mimic. Using a clean straight pin, poke each deposit circle site and transfer to the appropriate vial, capping each vial upon transferring. Follow the same procedure for the second piece of skin mimic. The cut-out pieces of treated skin mimic are then extracted with a solvent and the extract is analyzed and quantified. Add 50 µL of internal standard and 5 mL of 50:50 isopropyl alcohol:heptane to the cut-out pieces of skin mimic in the 20 mL glass vial. Cap the vial tightly and vortex at 1500 rpm (pulse) for 10 minutes. Transfer extract to autosampler vial. Gas chromatography analysis was conducted on Agilent 6890, or an equivalent device with a split/splitless capillary inlet system, flame ionization detector, and data system. A gas chromatography column of Agilent DB-1HT, 15 M x 0.25 mm ID, 0.10 µm film thickness or equivalent was used.

### V. Examples

### A. Examples 1 to 7

For Examples 2-7, personal cleansing compositions having structured cleansing phases are formed with different benefit phases Example 3 is according to the claims, the examples 1, 2 and 4 to 7 are comparative example. Compositional information with respect to Examples 1 to 7 can be found in Table 2. The cleansing phase for each of Examples 2-7 is prepared by adding water in a mixing vessel. Then the following ingredients are added with continuous mixing: sodium chloride, water soluble cationic polymer (guar hydroxypropyltrimonium chloride, N-Hance 3196 CG-17), laurylamidopropyl betaine, sodium trideceth sulfate, sodium tridecyl sulfate, ethoxylated tridecyl alcohol, Dissovine na2-S, sodium benzoate, and AQUPEC® SER W-300C. Adjust the pH by adding hydrogen peroxide (50% solution) to pH = 5.7 ± 0.2. Add methyl chloro isothiazolinone and methyl isothiazolinone, and mix until homogeneous. The benefit phase for Inventive Examples 2 is prepared by heating the lipids until they melt and mixing until homogeneous. For Inventive Examples 3-7, where the benefit phase contains a wax, the benefit phase is warmed and added to a warmed surfactant phase (∼70°C). The mixture is then mixed on a stand mixer until the composition is cooled to room temperature. The benefit phase for each of the Inventive Examples 2-7 is added into the surfactant phase through a SpeedMixer™ at a speed of 1,000 rpm for 60 seconds.

**Table 2**

| Ingredient / Property | Comparative Example 1 | Comparative Example 2 | Inventive Example 3 | Comparative Example 4 | Comparative Example 5 | Comparative Example 6 | Comparative Example 7 |
|---|---|---|---|---|---|---|---|
| Cleansing Phase (amts. by wt. % of cleansing phase) | | | | | | | |
| Distilled Water | 100 | Q.S. | Q.S. | Q.S. | Q.S. | Q.S. | Q.S. |
| Sodium Tridecyl Ether Sulfate | -- | 12.5 | 12.5 | 12.5 | 12.5 | 12.5 | 12.5 |
| Laurylamidopropyl Betaine | -- | 7.81 | 7.81 | 7.81 | 7.81 | 7.81 | 7.81 |
| Sodium Chloride | -- | 4.75 | 4.75 | 4.75 | 4.75 | 4.75 | 4.75 |
| Iconol TDA3-Ethoxylated Tridecyl Alcohol | -- | 1.00 | 1.00 | 1.00 | 1.00 | 1.00 | 1.00 |
| Water-soluble cationic polymer | -- | 0.42 | 0.42 | 0.42 | 0.42 | 0.42 | 0.42 |
| Sodium Benzoate, NF | -- | 0.28 | 0.28 | 0.28 | 0.28 | 0.28 | 0.28 |
| Methylchlorois othiazo-linone/methylis othiaxo-linone | -- | 0.037 | 0.037 | 0.037 | 0.037 | 0.037 | 0.037 |
| AQUPEC® SERW-300C | -- | 0.20 | 0.20 | 0.20 | 0.20 | 0.20 | 0.20 |
| Dissovine na2-s | -- | 0.15 | 0.15 | 0.15 | 0.15 | 0.15 | 0.15 |
| Hydrogen peroxide solution, 50% | -- | 0.005 | 0.005 | 0.005 | 0.005 | 0.005 | 0.005 |

| Benefit Phase (amts. by wt. % of benefit phase) | | | | | | | |
|---|---|---|---|---|---|---|---|
| RBD Soybean Oil | -- | 100 | -- | -- | -- | -- | -- |
| Hydrogenated Soybean Oil and Soy Oligomer Blend | -- | -- | 100 | -- | -- | -- | -- |
| Sucrose Polyester | -- | -- | -- | 80.0 | -- | -- | -- |
| Olivem 1000 | -- | -- | -- | 20.0 | -- | 20.0 | -- |
| Petrolatum | -- | -- | -- | -- | 87.0 | 66.7 | 83.3 |
| GlycerylMonooleate | -- | -- | -- | -- | 13.0 | 13.3 | 13.3 |
| Zinc Pyrithione | -- | -- | -- | -- | -- | -- | 3.3 |
| | | | | | | | |
| Cleansing phase : Benefit phase Ratio | -- | 85:15 | 85:15 | 85:15 | 85:15 | 85:15 | 85:15 |
| *in vitro* Deposition (µg/cm²) | 0 | 16 | 1037 | 130 | 361 | 426 | 333 |
| *in vivo* Chroma (3 hours after 3 treatments) | 16.9 | 17.8 | 18.1 | 17.8 | 18.5 | 18.4 | 18.5 |
| *in vitro* Chroma | 18.9 | 20.1 | 20.4 | 19.9 | 21.5 | 21.6 | 21.4 |
| *in vivo* Chroma (24 hours after 4 treatments | 17.4 | 17.6 | 17.8 | 17.7 | 18.0 | 17.8 | 18.0 |
| *in vivo* Chroma (3 hours after 5 treatments) | 17.1 | 17.9 | 18.4 | 18.0 | 18.5 | 18.5 | 18.3 |
| *in vivo* Chroma (3 hours after 14 treatments) | 17.5 | 18.0 | 18.9 | 18.2 | 18.6 | 18.5 | 18.6 |
| Corneometer (24 hours after 4 treatments) | 19.5 | 20.1 | 20.5 | 21.2 | 23.4 | 22.8 | 23.4 |
| Dry Skin Grade (3 hours after 3 treatments) | 3.1 | 2.6 | 2.0 | 2.4 | 1.0 | 0.9 | 1.2 |

Example 3 is according to the claims. The examples 1, 2 and 4 to 7 are comparative examples.

### B. Skin Chroma Values After Use of Compositions with Soy Oligomer and Soybean Oil

Table 3 provides four personal cleansing compositions including soybean oil and/or a soy oligomer. Compositional information with respect to Examples 8-11 can be found in Table 3. Examples 8-11 are formed in the same manner described for Inventive Examples 2-7. As shown, the compositions with the soy oligomer provided the highest skin chroma values.

**Table 3.**

| Ingredient / Property | Comparative Example 8 | Inventive Example 9 | Inventive Example 10 | Inventive Example 11 |
|---|---|---|---|---|
| Cleansing Phase (amts. by wt. % of cleansing phase) | | | | |
| Distilled Water | Q.S. | Q.S. | Q.S. | Q.S. |
| Sodium Tridecyl Ether Sulfate | 12.5 | 12.5 | 12.5 | 12.5 |
| Laurylamidopropyl Betaine | 7.81 | 7.81 | 7.81 | 7.81 |
| Sodium Chloride | 4.75 | 4.75 | 4.75 | 4.75 |
| Iconol TDA3-Ethoxylated Tridecyl Alcohol | 1.00 | 1.00 | 1.00 | 1.00 |
| Water-soluble cationic polymer | 0.42 | 0.42 | 0.42 | 0.42 |
| Sodium Benzoate, NF | 0.28 | 0.28 | 0.28 | 0.28 |
| Methylchloroisothiazo-linone/methylisothiaxo-linone | 0.037 | 0.037 | 0.037 | 0.037 |
| AQUPEC ® SER W-300C | 0.20 | 0.20 | 0.20 | 0.20 |
| Dissovine na2-s | 0.15 | 0.15 | 0.15 | 0.15 |
| Hydrogen peroxide solution, 50% | 0.005 | 0.005 | 0.005 | 0.005 |

| Benefit Phase (amts. by wt. % of benefit phase) | | | | |
|---|---|---|---|---|
| RBD Soybean Oil | 100 | 90 | 90 | 90 |
| Soy Oligomer | -- | 10 | 10 | 10 |
| | | | | |
| Cleansing phase : Benefit phase Ratio | 90:10 | 90:10 | 95:5 | 98:2 |
| *in vitro* Chroma | 20.4 | 22.19 | 21.31 | 21.16 |

Example 8 is a comparative example. The examples 9 to 11 are according to the claims.

### C. Skin Chroma Values After Use of Compositions with Soy Oligomer and SEFOSE®

Table 4 provides four personal cleansing compositions including SEFOSE® and/or a soy oligomer. Compositional information with respect to Examples 12-15 can be found in Table 4. Examples 12-15 are formed in the same manner described for Examples 2-7. As shown, the compositions with the soy oligomer provided the highest skin chroma values.

**Table 4.**

| Ingredient / Property | Comparative Example 12 | Inventive Example 13 | Inventive Example 14 | Inventive Example 15 |
|---|---|---|---|---|
| Cleansing Phase (amts. by wt. % of cleansing phase) | | | | |
| Distilled Water | Q.S. | Q.S. | Q.S. | Q.S. |
| Sodium Tridecyl Ether Sulfate | 12.5 | 12.5 | 12.5 | 12.5 |
| Laurylamidopropyl Betaine | 7.81 | 7.81 | 7.81 | 7.81 |
| Sodium Chloride | 4.75 | 4.75 | 4.75 | 4.75 |
| Iconol TDA3-Ethoxylated Tridecyl Alcohol | 1.00 | 1.00 | 1.00 | 1.00 |
| Water-soluble cationic polymer | 0.42 | 0.42 | 0.42 | 0.42 |
| Sodium Benzoate, NF | 0.28 | 0.28 | 0.28 | 0.28 |
| Methylchlorois othiazo-linone/methylis othiaxo-linone | 0.037 | 0.037 | 0.037 | 0.037 |
| AQUPEC® SER W-300C | 0.20 | 0.20 | 0.20 | 0.20 |
| Dissovine na2-s | 0.15 | 0.15 | 0.15 | 0.15 |
| Hydrogen peroxide solution, 50% | 0.005 | 0.005 | 0.005 | 0.005 |

| Benefit Phase (amts. by wt. % of benefit phase) | | | | |
|---|---|---|---|---|
| SEFOSE® 1618S | 100 | 90 | 95 | 97.5 |
| Soy Oligomer | -- | 10 | 5 | 2.5 |
| | | | | |
| Cleansing phase : Benefit phase Ratio | 90:10 | 90:10 | 90:10 | 90:10 |
| *in vitro* Chroma | 21.45 | 22.24 | 22.52 | 22.37 |

Example 12 is a comparative example. The examples 13 to 15 are according to the claims.

### D. Comparative Examples 16 to 22

For the comparative examples in table 5, personal cleansing compositions include non-structured cleansing phases formed with different benefit phases. Compositional information with respect to (comparative) examples 16 to 22 can be found in Table 5. As shown, the compositions with Polyquaternium 76 provided the highest skin chroma values. Further, as shown in Table 5, *in vitro* chroma values increase with increasing amounts of SEFOSE® 1618S. Comparative examples 16 to 22 are formed in the same manner described for Examples 2-7.

**Table 5.**

| Ingredient / Property | Compar. Ex. 1 | Compar. Ex. 16 | Compar. Ex 17 | Compar. Ex. 18 | Compar. Ex. 19 | Compar. Ex.20 | Compar. Ex. 21 | Compar. Ex. 22 |
|---|---|---|---|---|---|---|---|---|
| Distilled Water | 100 | Q.S. | Q.S. | Q.S. | Q.S. | Q.S. | Q.S. | Q.S. |
| Sodium Laureth Sulfate | -- | 14.22 | 14.22 | 14.22 | 14.22 | 14.22 | 14.22 | 14.22 |
| Sodium Lauryl Sulfate | -- | 5.47 | 5.47 | 5.47 | 5.47 | 5.47 | 5.47 | 5.47 |
| Citric Acid Anhydrous | -- | 0.33 | 0.33 | 0.33 | 0.33 | 0.33 | 0.33 | 0.33 |
| Cocamidopropyl Betaine | -- | 2.19 | 2.19 | 2.19 | 2.19 | 2.19 | 2.19 | 2.19 |
| Dis odium EDTA | -- | 0.22 | 0.22 | 0.22 | 0.22 | 0.22 | 0.22 | 0.22 |
| Sodium Benzoate | -- | 0.55 | 0.55 | 0.55 | 0.55 | 0.55 | 0.55 | 0.55 |
| Methylchlorois othiazo- linone | -- | 0.001 | 0.001 | 0.001 | 0.001 | 0.001 | 0.001 | 0.001 |
| Methylis othiaxolinone | -- | 0.0003 | 0.0003 | 0.0003 | 0.0003 | 0.0003 | 0.0003 | 0.0003 |
| Polyquatemium 76/AMT (95:5) | -- | -- | -- | -- | -- | 0.30 | 0.30 | 0.30 |
| SEFOSE® 1618S | -- | -- | 1.00 | 5.00 | 10.00 | 1.00 | 5.00 | 10.00 |
| | | | | | | | | |
| *in vitro* Chroma | 17.1 | 16.7 | 17.3 | 17.7 | 17.9 | 18.4 | 18.7 | 19.4 |

All of the examples in Table 5 above are comparative examples. The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

## Claims

1. A method of increasing skin chroma, the method comprising improving skin hydration by applying a multiphase personal cleansing composition comprising a benefit agent to the skin, said method comprising providing a rinse-off personal cleansing composition comprising a hydrophobic benefit agent, and
depositing 5 µg/cm² or more of the hydrophobic benefit agent on skin;
wherein the composition is multi-phase and comprises a cleansing phase and a benefit phase comprising the hydrophobic benefit agent and a soy oligomer; and
wherein the level of hydration is in accordance with one of the following: a Corneometer measurement of 0.3 Corneometer units or more above a water control after treatment of at least once a day for at least 4 consecutive days when measured at 24 hours after the last treatment in accordance with the Corneometer Testing Method; a dry skin grade of 0.4 units or more above a water control after treatment of at least once a day for at least 3 consecutive days when measured at 3 hours after the last treatment in accordance with the Dry Skin Grade Method; or a combination thereof.

2. The method of the preceding claim, wherein an increase in skin chroma of 0.2, preferably 0.3, more preferably 0.4, or most preferably 0.6, above the water control is measureable after treatment at least once a day for at least 4 consecutive days when measured at 24 hours after the last treatment in accordance with the Chroma Test Method.

3. The method of claim 1, wherein an increase in skin chroma of 0.8, preferably 0.9, more preferably 1.2, or most preferably 1.4, above the water control is measureable after treatment at least once a day for at least 5 consecutive days when measured at 3 hours after the last treatment in accordance with the Chroma Test Method.

4. The method of claim 1, wherein an increase in skin chroma of 0.5, preferably 0.7, more preferably 1.0, or most preferably 1.1, above the water control is measureable after treatment at least once a day for at least 14 consecutive days when measured at 3 hours after the last treatment in accordance with the Chroma Test Method.

5. The method of any preceding claim, wherein the cleansing phase and benefit phase are visually distinct.

6. The method of any preceding claim, wherein the cleansing phase is structured.

7. The method of any preceding claim, wherein the deposition is 16 µg or more, preferably 130 µg or more, more preferably 333 µm or more, or most preferably 426 µg or more.

8. The method of any preceding claim, wherein the cleansing phase to benefit phase ratio is 97.5:2.5, preferably 95:5, more preferably 90:10, or even more preferably 85:15.

9. The method of any preceding claim, wherein the hydrophobic benefit agent exhibits a total surface energy of about 20 mJ/m² to about 30 mJ/m² and a change in heat of fusion of about 50 J/g or less.

10. The method of any of claims 2-9, wherein the multi-phase personal cleansing composition comprises a cleansing phase comprising sodium trideceth (n) sulfate or sodium laureth(n) sulfate and the benefit phase comprises the hydrophobic benefit agent.

11. The method of any preceding claim, wherein the hydrophobic benefit agent comprises unsaturated soybean oil, petrolatum, mineral oil, sucrose polyester, glyceryl monooleate, fatty esters, fatty alcohols, or a combination thereof.

12. The method of any preceding claim, wherein the composition is free of a pigment.

## Patentansprüche

1. Verfahren zum Erhöhen der Hautchrominanz, wobei das Verfahren Folgendes umfasst
Verbessern der Hautdurchfeuchtung durch Auftragen einer Mehrphasenkörperreinigungszusammensetzung, die einen Pflegewirkstoff umfasst, auf die Haut, wobei das Verfahren Folgendes umfasst
Bereitstellen einer abwaschbaren Körperreinigungszusammensetzung, die einen hydrophoben Pflegewirkstoff umfasst, und
Aufbringen von 5 µg/cm² oder mehr des hydrophoben Pflegewirkstoffs auf die Haut;
wobei die Zusammensetzung mehrphasig ist und eine Reinigungsphase und eine Pflegephase umfasst, die den hydrophoben Pflegewirkstoff und ein Soja-Oligomer umfasst; und
wobei das Durchfeuchtungsmaß einem der Folgenden entspricht: einer Corneometer-Messung von 0,3 Corneometer-Einheiten oder mehr über einer Wasserkontrolle nach der Behandlung mindestens einmal täglich für mindestens 4 aufeinanderfolgende Tage bei Messung 24 Stunden nach der letzten Behandlung gemäß dem Corneometer-Prüfverfahren; einem Hauttrockenheitsgrad von 0,4 Einheiten oder mehr über einer Wasserkontrolle nach der Behandlung mindestens einmal täglich für mindestens 3 aufeinanderfolgende Tage bei Messung 3 Stunden nach der letzten Behandlung gemäß dem Hauttrockenheitsgradverfahren; oder einer Kombination davon.

2. Verfahren nach dem vorstehenden Anspruch, wobei eine Steigerung der Hautchrominanz von 0,2, vorzugsweise 0,3, mehr bevorzugt 0,4 oder am meisten bevorzugt 0,6 über der Wasserkontrolle nach der Behandlung mindestens einmal täglich für mindestens 4 aufeinanderfolgende Tage bei Messung 24 Stunden nach der letzten Behandlung gemäß dem Chrominanzprüfverfahren messbar ist.

3. Verfahren nach Anspruch 1, wobei eine Steigerung der Hautchrominanz von 0,8, vorzugsweise 0,9, mehr bevorzugt 1,2 oder am meisten bevorzugt 1,4 über der Wasserkontrolle nach der Behandlung mindestens einmal täglich für mindestens 5 aufeinanderfolgende Tage bei Messung 3 Stunden nach der letzten Behandlung gemäß dem Chrominanzprüfverfahren messbar ist.

4. Verfahren nach Anspruch 1, wobei eine Steigerung der Hautchrominanz von 0,5, vorzugsweise 0,7, mehr bevorzugt 1,0 oder am meisten bevorzugt 1,1 über der Wasserkontrolle nach der Behandlung mindestens einmal täglich für mindestens 14 aufeinanderfolgende Tage bei Messung 3 Stunden nach der letzten Behandlung gemäß dem Chrominanzprüfverfahren messbar ist.

5. Verfahren nach einem der vorstehenden Ansprüche, wobei die Reinigungsphase und die Pflegephase optisch voneinander unterscheidbar sind.

6. Verfahren nach einem der vorstehenden Ansprüche, wobei die Reinigungsphase strukturiert ist.

7. Verfahren nach einem der vorstehenden Ansprüche, wobei die Aufbringung 16 µg oder mehr, vorzugsweise 130 µg oder mehr, mehr bevorzugt 333 µm oder mehr oder am meisten bevorzugt 426 µg oder mehr beträgt.

8. Verfahren nach einem der vorstehenden Ansprüche, wobei das Verhältnis der Reinigungsphase zur Pflegephase 97,5:2,5, vorzugsweise 95:5, mehr bevorzugt 90:10 oder noch mehr bevorzugt 85:15 beträgt.

9. Verfahren nach einem der vorstehenden Ansprüche, wobei der hydrophobe Pflegewirkstoff eine Gesamtoberflächenenergie von etwa 20 mJ/m² bis etwa 30 mJ/m² und eine Veränderung der Fusionswärme von etwa 50 J/g oder weniger aufweist.

10. Verfahren nach einem der Ansprüche 2 bis 9, wobei die Mehrphasenkörperreinigungszusammensetzung eine Reinigungsphase umfasst, die Natriumtrideceth(n)sulfat oder Natriumlaureth(n)sulfat umfasst, und die Pflegephase den hydrophoben Pflegewirkstoff umfasst.

11. Verfahren nach einem der vorstehenden Ansprüche, wobei der hydrophobe Pflegewirkstoff ungesättigtes Sojaöl, Petrolatum, Mineralöl, Saccharosepolyester, Glycerylmonooleat, Fettester, Fettalkohole oder eine Kombination davon umfasst.

12. Verfahren nach einem der vorstehenden Ansprüche, wobei die Zusammensetzung frei von einem Pigment ist.

## Revendications

1. Procédé d'augmentation de l'intensité chromatique de la peau, le procédé comprenant
l'amélioration de l'hydratation de la peau en appliquant une composition pour la toilette polyphasique
comprenant un agent bénéfique sur la peau ledit procédé comprenant
la fourniture d'une composition pour la toilette à éliminer par rinçage comprenant un agent bénéfique hydrophobe, et
le dépôt de 5 µg/cm² ou plus de l'agent bénéfique hydrophobe sur la peau ; dans lequel la composition est polyphasique et comprend une phase nettoyante et une phase bénéfique comprenant l'agent bénéfique hydrophobe et un oligomère de soja ; et
dans lequel le niveau d'hydratation est en conformité avec l'une des valeurs suivantes : une mesure de cornéomètre de 0,3 unité de cornéomètre ou plus au-dessus d'un témoin eau après un traitement d'au moins une fois par jour pendant au moins 4 jours consécutifs lorsqu'on mesure 24 heures après le dernier traitement conformément au procédé d'essai par cornéomètre ; une qualité de peau sèche de 0,4 unité ou plus au-dessus d'un témoin eau après un traitement d'au moins une fois par jour pendant au moins 3 jours consécutifs lorsqu'on mesure 3 heures après le dernier traitement conformément au procédé de qualité de peau sèche ; ou une combinaison de celles-ci.

2. Procédé selon la revendication précédente, dans lequel une augmentation d'intensité chromatique de la peau de 0,2, de préférence 0,3, plus préférablement 0,4, ou le plus préférablement 0,6, au-dessus du témoin eau est mesurable après un traitement d'au moins une fois par jour pendant au moins 4 jours consécutifs lorsqu'on mesure 24 heures après le dernier traitement conformément au procédé de test d'intensité chromatique.

3. Procédé selon la revendication 1, dans lequel une augmentation d'intensité chromatique de la peau de 0,8, de préférence 0,9, plus préférablement 1,2, ou le plus préférablement 1,4, au-dessus du témoin eau est mesurable après un traitement d'au moins une fois par jour pendant au moins 5 jours consécutifs lorsqu'on mesure 3 heures après le dernier traitement conformément au procédé de test d'intensité chromatique.

4. Procédé selon la revendication 1, dans lequel une augmentation d'intensité chromatique de la peau de 0,5, de préférence 0,7, plus préférablement 1,0, ou le plus préférablement 1,1, au-dessus du témoin eau est mesurable après un traitement d'au moins une fois par jour pendant au moins 14 jours consécutifs lorsqu'on mesure 3 heures après le dernier traitement conformément au procédé de test d'intensité chromatique.

5. Procédé selon l'une quelconque des revendications précédentes, dans lequel la phase nettoyante et la phase bénéfique sont visuellement distinctes.

6. Procédé selon l'une quelconque des revendications précédentes, dans lequel la phase nettoyante est structurée.

7. Procédé selon l'une quelconque des revendications précédentes, dans lequel le dépôt est de 16 µg ou plus, de préférence 130 µg ou plus, plus préférablement 333 µg ou plus, ou le plus préférablement 426 µg ou plus.

8. Procédé selon l'une quelconque des revendications précédentes, dans lequel le rapport de la phase nettoyante à la phase bénéfique est de 97,5:2,5, de préférence 95:5, plus préférablement 90:10, ou même plus préférablement 85:15.

9. Procédé selon l'une quelconque des revendications précédentes, dans lequel l'agent bénéfique hydrophobe présente une énergie de surface totale d'environ 20 mJ/m² à environ 30 mJ/m² et un changement de chaleur de fusion d'environ 50 J/g ou moins.

10. Procédé selon l'une quelconque des revendications 2 à 9, dans lequel la composition pour la toilette polyphasique comprend une phase nettoyante comprenant du tridéceth(n) sulfate de sodium ou du laureth(n) sulfate de sodium et la phase bénéfique comprend l'agent bénéfique hydrophobe.

11. Procédé selon l'une quelconque des revendications précédentes, dans lequel l'agent bénéfique hydrophobe comprend de l'huile de soja insaturée, de la vaseline, une huile minérale, du polyester de saccharose, du monooléate de glycéryle, des esters gras, des alcools gras, ou une combinaison de ceux-ci.

12. Procédé selon l'une quelconque des revendications précédentes, dans lequel la composition est exempte de pigment.
